# EUROPEAN PATENT APPLICATION

(11) **EP 4 140 468 A2**
(43) Date of publication of application: **01.03.2023**
(21) Application number: 21729907.2
(22) Date of filing: 26.04.2021
(51) Int. Cl.: A61K 8/02, A61K 8/73, A61K 8/81, A61K 8/85, A61K 8/86, A61Q 13/00

(54) **SELF-ADHERENT PATCHES OF POLYMER FIBRES FOR THE CONTROLLED RELEASE OF PERFUME**

(30) Priority: 24.04.2020 ES 202030345
(71) Applicant: Bioinicia, S.L., 46980 Paterna (Valencia) (ES); Perfumes Loewe, S.A., 28034 Madrid (ES); Consejo Superior de Investigaciones Científicas (CSIC), 28006 Madrid (ES)
(72) Inventor: LAGARÓN CABELLO, José María, 28006 Madrid (ES); TENO DÍAZ, Jorge, 46988 Paterna Valencia (ES); PARDO FIGUEREZ, María De Las Mercedes, 46988 Paterna Valencia (ES); MARTÍN DE BUSTAMANTE ENRÍQUEZ, Lourdes, 28034 Madrid (ES)
(74) Representative: Pons
(86) International application number: PCT/ES2021/070276
(87) International publication number: WO 2021/214370

(57) **Abstract**

The present invention relates to multilayer patches of polymer fibres as perfume-releasing platforms with are of great interest for the cosmetics and pharmaceutical industry, as they enable the user to use perfumes without having to be in direct contact with them, preventing potential allergies and/or adverse effects. The invention also relates to the methods for the preparation of said patches, which use electro-hydrodynamic and aero-hydrodynamic processing techniques.

## Description

The present invention falls within the area of polymer materials based on fibres and ultrafine particles applied to the cosmetics and pharmaceutical sector, and it relates to a method and the application thereof for manufacturing fibre patches as a perfume-release platform by means of electro-hydrodynamic and/or aero-hydrodynamic processing techniques.

### BACKGROUND OF THE INVENTION

Lotions and perfumes are commonly applied to the skin of users. These substances are formulated so that the scent is maintained for as long as possible on the area of application, in fact, it is difficult for a user to apply the optimal amount of perfume so that the scent is maintained throughout the day, since the scent of the perfume is practically completely dissipated by the end of the day. For a long-lasting scent, current perfumes would probably have to be highly concentrated, which would lead to an excessive cost of the perfumes. Furthermore, the use of perfumes in direct contact with the skin can cause some irritation, dermatitis or even allergies in some cases, especially in high concentrations.

Both the duration of the scent and the possible negative effects that it can cause to the skin of the users are two of the problems to be solved within the cosmetics industry. To this end, one of the possible solutions is the use of scented patches that can adhere to the skin of any user maintaining the same perfume intensity for 24 hours.

Two patented designs of scented materials have been found, U.S. Pat. No. 2013/0078421 A1 by Desiderio et al. and U.S. Pat. No. 6,723,671 by Zolotarsky. In the case of U.S. Patent No. 2013/0078421 A1 by Desiderio et al., the perfume is encapsulated in microcapsules within the adhesive and is released by pressing the patch. Although it has certain advantages, this type of patch requires users to be aware of pressing the patch to release the perfume, which can become a nuisance. In addition, in patches of this type, the perfume is released directly to the skin so it does not solve the problem of possible reactions or allergies.

Furthermore, the scented patches described have the additional drawback of becoming plastic waste once their use is completed. This is because the materials used contain different chemical substances and polymers that are not soluble in polar or biodegradable solvents, so if the user does not manage their waste properly, the patch can end up polluting the ecosystem. In contrast, the patches proposed in the present patent are preferably manufactured with materials soluble in polar and/or biodegradable solvents, which provides added value to the invention.

### DESCRIPTION OF THE INVENTION

The present invention proposes a methodology to generate a multilayer patch manufactured in a monoaxial manner, coaxial manner, by co-deposition or by the layer by layer apprach. The patch of the invention is made of micrometric, submicrometric and also nanometric fibres, and incorporates one or more scents therein which provide an effect of persistent perfumed patch, whilst adheres to the skin of any body part, such as the neck.

The possible patch structures are described below:
- Continuously manufactured multilayer, comprising at least two layers, one that is an inner layer made by mixing or emulsion that is in contact with the skin and that is hydrophilic to promote adhesion; and another outer layer that generally storages the perfume, also made by mixing or by emulsion of the same or of a material other than the inner layer, and applied continuously on it, and the function of which is to release the perfume in a controlled manner and/or to provide resistance to humidity. In addition, one or more intermediate layers can be included between the outer layer and the inner layer, also continuously, which may or may not contain perfume and which promote adhesion between the layers and/or enable perfume to be released in a controlled manner. This process may or may not lead to an additional step of heat treatment with or without pressure using any technique available for this purpose, with the aim of improving the surface touch, printing, interlayer adhesion and to reduce thickness. Preferably, it will undergo a hot calendering process at low temperature to prevent the loss and/or balance of the perfume.
- Multilayer manufactured by lamination of individual layers, comprising at least two layers, one that is an inner layer made by mixing or emulsion that is in contact with the skin and that is hydrophilic to promote adhesion; and another outer layer that generally storages the perfume, also made by mixing or by emulsion of the same or of a different material other than the inner layer, The function of this layer is to release the perfume in a controlled manner and/or to provide resistance to humidity. In addition, one or more intermediate layers can be included between the outer layer and the inner layer, which may or may not contain perfume and which promote adhesion between the layers and/or enable perfume to be released in a controlled manner. The lamination of the different layers can be carried out by any industrial lamination method with or without pressure, preferably by calendering at low temperature to prevent the loss and/or balance of the perfume.
- Continuously manufactured and laminated multilayer. This possible structure is the combination of the preceding two structures. In other words, a continuous multilayer patch of several layers can be carried out separately and then a laminating process thereof can be carried out to promote the attachment between the layers, providing a smoother texture, improving interlayer adhesion and/or reducing thickness. Lamination can be carried out by any industrial lamination method with or without pressure, preferably by calendering at low temperatures to prevent the loss and/or balance of the perfume.

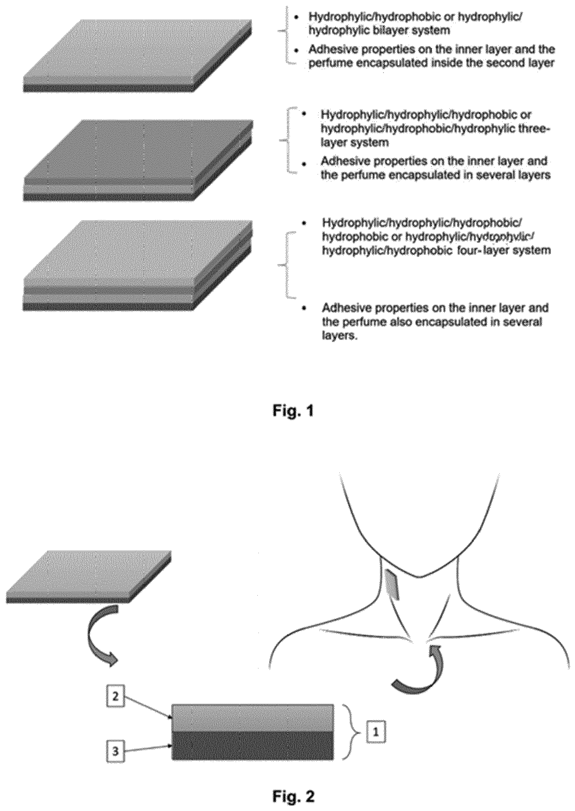

In a general manner, one or more of the layers would contain the perfume, while the other layer in contact with the skin would be hydrophilic and would have self-adhesive properties (see Fig. 1 for different configurations by way of example). Fig. 2 shows a basic example of the shape and use of a bilayer patch.

Therefore, a first aspect of the present invention relates to a perfume-releasing patch characterised in that it comprises at least:
i) An inner layer that is in contact with the user's skin to which it adheres, characterised in that it is made up of fibres of a polymer A. By being made of very fine fibres, the great surface-to-volume ratio of these fibres generates local dissolution and strong adhesion without the need to add other adhesive substances. This layer must have a surface density of at least 0.1 g/m², more preferably between 20 and 300 g/m², even more preferably between 30 and 175 g/m²;
ii) a series of between 0 and 1000 consecutive intermediate layers deposited on the inner layer, characterised in that they are independently made up of fibres of a polymer A or B, or a mixture of both, which optionally contain the encapsulated perfume, and that have a surface density of at least 0.1 g/m²; more preferably between 2 and 1000 g/m²;
iii) an outer layer optionally containing the encapsulated perfume, characterised by being made of fibres of a polymer B and having a surface density of at least 0.1 g/m²; more preferably between 0.5 and 1000 g/m².

The polymers that are used in the patch of the invention are preferably water-soluble and/or biodegradable polymers.

In the present invention, the surface density normally expressed in g/m² for each of the layers is calculated by weighing a sample with known dimensions. Subsequently, the weight is divided by the surface of the sample. This process is carried out with at least 5 samples of each layer in order to thus obtain a mean surface density value for the entire layer.

In the present invention, the term "encapsulation" refers to the incorporation of the perfume both inside the fibres of the polymers that make each of the layers of the patch that contain it, forming a separate core-shell phase; and also constituting a physical mixture with the polymer material of the fibre, including what is known as a solid dispersion; the perfume therefore being able to be found both inside and on the surface of the fibres, or even in the interstitial spaces between them.

In a preferred embodiment, the polymer A is a hydrophilic polymer, soluble in water, alcohols and/or a mixtures thereof, and more preferably it is soluble in water, and it is selected, without limitation, from the following: polyethylene oxide (PEO) and derivatives thereof as non-ionic water-soluble resins (polyox WSR), polyvinylpyrrolidone (PVP) and the copolymers thereof, polyvinyl alcohols (PVOH) and the copolymers thereof with ethylene (EVOH), polyacrylates (PAC), polyacrylic acid (PAA), water-soluble polyacronitriles (PAN), lignin and derivatives such as sulfonated lignin (SL), acrylic/methacrylic ester polymers, polysaccharides and derivatives, such as for example pullulan, hyaluronic acid, alginate, tragacanth, carrageenan, chitin and derivatives such as chitosan, celluloses such as ethylcellulose, hydroxypropylcellulose, methylcellulose, or hydroxypropylmethylcellulose, gluocogen, starch and polymers derived from the same such as thermoplastic starch (TPS), pectin, guar, xanthan, fructosan or gellan among others, proteins and derivatives, such as collagen, gelatin, soy protein, whey protein, zein, gluten, casein, or lectins among others, thiolated polymers, polyanhydrides, and PAA polyethylene glycol copolymers (PAA-co-PEG), as well as mixtures of any of the above, or any of the above mixed with additives such as plasticisers, surfactants, antioxidants, dyes, etc.

In a more preferred embodiment, the polymer of the inner layer that is in contact with the skin is selected from PVP, PEO, PVOH, polyacrylates, zeins, gluten derivatives, cellulosic materials or combinations thereof. In an even more preferred embodiment, the inner layer comprises an emulsified mixture of PEO and PVP that also contains acrylates, zein, gluten derivatives, ethyl cellulose or a mixture thereof.

In a preferred embodiment, the polymer A of the inner layer is polyethylene oxide.

In another preferred embodiment, the polymer A of the inner layer is polyvinylpyrrolidone.

In another preferred embodiment, the polymer A of the inner layer is a mixture of polyvinylpyrrolidone and polyacrylate.

In another preferred embodiment, the polymer A of the inner layer is a mixture of polyethylene oxide, polyvinylpyrrolidone and ethyl cellulose.

In another preferred embodiment, the polymer B is a polymer soluble in organic solvents, that are selected, without limitation, from non-water-soluble proteins such as keratin, waxes or paraffins, polyhydroxyalkanoates (PHA) such as PHB, PHV, medium chain length PHA (mcl-PHA), and all the possible copolymers thereof such as PHBV among others, poly-ε-caprolactone (PCL) and all the copolymers thereof such as PEG-PCL and PCLA, polylactic acid (PLA), all the copolymers thereof such as PGLA, polyphosphazenes, polyorthoesters, polyesters obtained starting from natural precursors such as polytrimethylene terephthalate (PTT), polybutylene terephthalate (PBT), polybutylene succinate (PBS), and all the possible copolymers thereof such as poly(butylene adipate-co-terephthalate) (PBAT), among others, as well as other non-biodegradable polymers such as: polyolefins of which ethylene co-polymers can be highlighted, such as polyethylene-co-vinyl acetate (EVA), polyethylene terephthalate (PET) and copolymers thereof, silicones, polyesters, polyurethanes (PURs), polysulfones, halogenated polymers such as polyvinylidene fluoride (PVDF) or polyvinylidene chloride (PVDC), polycarbonates, acrylonitrile butadiene styrene, latex, and polyamides such as 6 Nylon 6, Nylon 66 or Nylon 69, as well as mixtures of any of the above, or any of the above mixed with additives such as plasticisers, surfactants, antioxidants, dyes, etc.

In a more preferred embodiment, the outer layer comprises a polymer B that is selected from poly-ε-caprolactone (PCL), poly-ε-caprolactone copolymers, polylactic acid (PLA), and polyhydroxyalkanoates. In a more preferred embodiment, the polymer of the outer layer is poly-ε-caprolactone, poly-ε-caprolactone copolymers, or any of the mixtures thereof, due to the high biodegradability and low melting point thereof.

In the present invention, the term "polymer" refers to macromolecular materials both in a pure ex-reactor state, as well as additives and post-processed materials in commercial formulas typically used by chemical industries, commonly called plastic grades. Process additives, promoters of biodegradability or promoters that confer stability, other type of additives of the "filler" type, either in micro-, submicro- or nanometric form, can be additionally added to any of the polymers or plastic grades to improve the physicochemical properties or the retention capacity and the controlled release of the perfume. Such additives can be chemicals type, fibres, sheets or particles.

In another preferred embodiment, the patch of the invention comprises intermediate layers located between the inner layer and the outer layer, that confer better adhesion to the patch between these layers. In an even more preferred embodiment, these intermediate layers contain the same perfume as the outer layer, in such a way that the release of the perfume is enhanced.

In an even more preferred embodiment, the polymer of the intermediate layers is independently selected from poly-ε-caprolactone, poly-ε-caprolactone copolymers, polyvinylpyrrolidone, polyethylene oxide and ethyl cellulose, or any of the mixtures thereof.

In a preferred embodiment, in addition to the perfume, different bioactive components with a cosmetic and pharmaceutical, functional or regenerative effect on the skin can be added to any of the layers of the patch to improve the impact of the patch during use. The bioactive components that can be added will be selected, without limitation, from: hyaluronic acids, vitamins, aloe vera, liposomes, antioxidants, anti-aging agents, anti-wrinkle agents, cell regenerating agents, retinol, etc. In an even more preferred embodiment, the bioactive agents will have no organoleptic impact and will be preferentially added to the inner layer that is in direct contact with the skin.

In a preferred embodiment, additives can be added to the intermediate layers and to the outer layer to generate extra properties such as oleophobic, amphiphobic and/or superhydrophobic properties, have colour by adding pigments, or even using some type of polarised polymer so that the colour varies with exposure to light. Also, some type of logo, pictograph or image, either multi-colour or single-colour, can be printed on these layers using any type of ink or any printing or stamping process, which do not alter the properties of the perfume. For this, water-based inks, oil or some organic solvent devoid of its own odour can be used. In the latter case, as a more preferred option, the inks used can be eco-solvents.

The patch proposed in this document can be presented in any shape or flat pattern, carried out by any conventional cutting method, either manual, using a die-cutting system, or laser cutting.

As discussed above, multilayer patches can be continuously manufactured by depositing each layer on top of the preceding one, or be separately manufactured, attaching them by means of any lamination method, or a combination of both. Regarding lamination, it can be carried out by any lamination method with or without pressure, preferably by calendering at low temperature.

Calendering can be carried out using two or more rollers with or without pressure, wherein at least one of them can be at the desired temperature, or it may be the case wherein all the rollers are at the desired temperature. In this step, the rollers can have a certain shape on the surface, thus giving the patches a texture and shape.

The calendering of the produced layers can be carried out in such a way that the inner layer is in contact with the heating roller, or, on the contrary, it is the last layer, which is the one in contact with the roller that is at the required temperature.

In another preferred embodiment, the different layers can be manufactured continuously one on top of the preceding one and then a heat treatment process can be carried out with or without pressure, preferably by calendering at low temperature, to ensure adhesion between layers, give the patch a smoother texture, and be able to reduce the thickness.

In another preferred embodiment, the perfume incorporated in the intermediate layers and/or in the outer layer of the patch of the invention can be selected, without limitation, from the following families, or combination thereof:
- Floral. Defined by those where the scent of flowers is predominant. The most used scents are from flowers such as rose, jasmine, peony, gardenia, tuberose, lily-of-the-valley, freesia, magnolia, geranium or mimosa.
- Fruity. The composition thereof is distinguished by the notes of ripe fruit; mango, ripe fig, peach, pear, apple, raspberry and other summery delights. These scents are usually complemented by floral scents and wood nuances, musks, amber or vanilla.
- Aquatic. They have fresh, subtle notes, with a scent of cleanliness. This family usually contains mainly Calone, which is a synthetic essence that provides a marine note.
- Green. They have a smell which evokes freshly cut grass, plant stems covered with dew, crisp leaves or pine needles in the forest air. They usually contain notes of pine, grass, petitgrain, mint, lily-of-the-valley, and especially galbanum, which brings freshness and also surprising depth.
- Aromatic. They stand out for notes of aromatic herbs; lavender, geranium, basil, cumin, rosemary and sage. These scents are complemented by woody notes. Sometimes, aromatic fragrances include nuances of moss, patchouli and balsamic notes, which confer warmth to the composition.
- Fougère. This family of perfumes contains lavender and oak moss. They also usually have notes of geranium, vetiver, bergamot and coumarin.
- Citrus. The compositions thereof are based on lemon, orange, bergamot, grapefruit and tangerine, and may also contain aromatic, floral and woody notes.
- Chypre. The perfumes of this family (or Chypre) are warm, dry and deep. Usually, the main scent is an accord of oak moss, bergamot, patchouli and laudanum. This scent is sometimes complemented by flowery, fruity or woody nuances.
- Woody. The composition of these perfumes focusses on the scents of sandalwood, cedar, oud, patchouli and vetiver. Woody fragrances also usually include citric, fruity, spicy, herbal or musky notes.
- Oriental. This family of perfumes contains ingredients such as vanilla and tonka bean. They also usually contain woody notes, especially sandalwood, often accompanied by spices and exotic flowers.
- Musk. The composition of this family of perfumes focusses on the different varieties of musk, complemented by rose, jasmine, cananga flower and lily flowery notes. They also usually contain woody notes and amber nuances.
- Leather. They are usually very intense, sensual, warm and sweet fragrances. They mainly combine woody, tobacco, amber and musk notes. They are also usually accompanied by vanilla, ginger, cocoa, liquorice, and spicy scents such as clove, pepper, and cardamom.

In another preferred embodiment, one or more perfumes can be incorporated into the intermediate layers of the patch of the invention and the same or another type of perfume can be added to the outer layer, or even as many layers as the perfumes to be added can be added, in such a way that the combination of both layers causes the release of a pleasant and intense odour. When these intermediate layers incorporate perfume, the outer layer may or may not contain an encapsulated perfume.

Regarding the manufacture of each of the layers that make up the patch of the invention, they are preferably carried out by means of any of the known electro-hydrodynamic and aero-hydrodynamic techniques for obtaining fibres, such as electrospinning, electrohydrodynamic direct writing, *solution blow spinning, electrospraying, solution blow spraying,* electrospraying assisted by pressurised gas or combination and/or variant of both. Nevertheless, any other method for obtaining fibres may also be used, such as centrifugal jet spinning or the combination of this and those previously mentioned. Electro-hydrodynamic and aero-hydrodynamic techniques are based on the formation of polymer micro- or nanofibres at room temperature or lower, from a polymer solution to which an electric field or gas pressure is applied. The fact that it is used in the form of a solution provides great versatility, since it enables various substances (perfume) to be incorporated into the solution itself. At the same time, given that the processability thereof occurs at room temperature, it prevents certain problems such as the degradation of the essential oil of the perfume.

With these techniques and the polymers mentioned above, in the present invention the perfume is incorporated in such a way that the release is controlled, so that it has a greater intensity and persistence. To carry out this incorporation, techniques are used which include, without limitation: core-shell technology, co-deposition, direct mixing, emulsion techniques, pre-encapsulation in particles, or layer by layer deposition, etc.

In the present invention, core-shell technology is used in the case of electrospinning and solution blow spinning, making use of a concentric nozzle through which the perfume with or without the polymers A or B is supplied through the inner tube, while the encapsulating agent, in this case the polymer selected to prepare the corresponding layer, is passed through the outer tube. This gives rise to tubular fibres inside of which the perfume is stored. In this case of using water-soluble biodegradable polymers, as they degrade, the controlled release of perfume occurs. If non-water-soluble biodegradable polymers are used, the perfume molecules spread through the wall of the fibres, thus controlling the releasing process.

In the present invention, co-deposition consists of a deposition method in which two injectors simultaneously deposit, for example, on one hand, the solution with the perfume and, on the other, the polymer solution. This technique is used when the perfume is not compatible in solution with the polymer of interest. It is also used by depositing two solutions that contain the perfume, but with different types of polymers, thus generating different fibre sizes and morphologies within the same membrane, and therefore a different perfume release profile. Co-deposition of perfume and fibres of the encapsulating agent, of particles and/or of fibres and/or of a mixture of the two can therefore be carried out. In addition, simultaneous electrospinning enables various properties to be combined within the same membrane.

In the present invention, the direct mixture can be, without limitation, of the encapsulated perfume and the encapsulating agent or of a suspension of particles containing the pre-encapsulated perfume and the encapsulating agent, by means of monoaxial electrospinning, giving rise to cylindrical fibres in which the perfume is embedded and dispersed within the fibre. This mixture can be a homogeneous solution or a heterogeneous suspension.

In the present invention, the emulsion techniques refer to any emulsion of, without limitation, solvents or components, which give rise to encapsulation with several phases and which are manufactured by the processes known as electrohydrodynamic or aero-hydrodynamic emulsion processing. An emulsion is a dispersion of a liquid (dispersed phase) in the form of very small particles within another liquid (continuous phase) with which it is generally immiscible. Emulsions can be direct, inverse or multiple. Direct emulsions are those in which the dispersed phase is a lipophilic substance and the continuous phase is hydrophilic. These emulsions are often called L/H or O/W. On the other hand, inverse emulsions, are those in which the dispersed phase is a hydrophilic substance and the continuous phase is lipophilic. These emulsions are often called the abbreviation H/L or W/O. Multiple emulsions are those that, as a dispersed phase, contain an inverse emulsion and the continuous phase is an aqueous liquid. These emulsions are known as H/L/H or W/O/W. Emulsions can also be formulated by means of the so-called Pickering emulsions that use particles to separate the phases and by means of any other type of emulsion technology. As such, the perfume is encapsulated within the fibres in the organic phase and the release also occurs in a controlled manner.

In the present invention, particle pre-encapsulation consists of obtaining fibres that are charged or mixed in the event of co-deposition, with micro- or nanoparticles in which the perfume has been previously encapsulated. For this, any encapsulation method is used that produces particles such as, and not limited to, *electrospray, air-assisted electrospray (EAPG), coacervation, emulsion-evaporation*/*emulsion-extraction, hot melt, interfacial polycondensation, complexing, gelling, fluidised bed, atomisation, lyophilisation, extrusion, electrostatic droplet generation, supercritical fluids, TROMS, etc. and mixtures thereof.* These perfumed particles are typically added, in the case of direct mixing, to a solution of the selected polymer, such that, after any of the aforementioned processes, fibres with particles inside are obtained. In this case, the method of controlled release of the perfume is carried out both by degradation of the particles and fibres and by diffusion through the particles and the fibre, or it may even be the case where the two release mechanisms occur simultaneously.

In the present invention, the layer-by-layer deposition method consists of the use of a system in which the layers are deposited sequentially within the same process. In this manner, initially one of the layers is electrospun until reaching the desired thickness and then the second layer is electrospun on top of the first, continuously obtaining a multilayer system *in situ.*

Therefore, a second aspect of the invention relates to obtaining a perfume-emitting patch comprising the following steps:
a) Preparation of the inner layer starting from a solution of the polymer A at a concentration between 0.01 and 98% by weight, wherein the emitter voltage used is between 0.01 and 500kV and a collector voltage is between 0 kV and -500kV, with a flow rate between 0.0001 and 1,000,000 ml/h, at a temperature between 1°C and 100°C and a relative humidity between 0% and 100%;
b) Preparation of the intermediate layers located between the inner layer and the outer layer starting from a solution of the polymer A or B at a concentration between 0.01 and 98% by weight, and optionally of a perfume in a concentration between 0 and 98% by weight, wherein the emitter voltage used is between 0.01 kV and 500kV and the collector voltage is between 0kV and -500kV, with a flow rate between 0.0001 and 1,000,000 ml/h, at a temperature between 1°C and 100°C and a relative humidity between 0% and 100%;
c) Preparation of the outer layer starting from a solution of the polymer B at a concentration between 0.01 and 98% by weight, and optionally of a perfume in a concentration between 0 and 98% by weight, wherein the emitter voltage used is between 0.01kV and 500kV and the collector voltage is between 0.01kV and - 500kV, with a flow rate between 0.0001 and 1,000,000 ml/h, at a temperature between 1°C and 100°C and a relative humidity between 0% and 100%;
d) Processing of the layers produced either continuously or separately in steps (a), (b), and (c) by means of calendering in a speed range between 0.001 and 100,000 rpm and a temperature between 5 and 300°C.

In a preferred embodiment, the method comprises as many additional steps (b) for preparing hydrophilic or hydrophobic layers as intermediate layers that are arranged between the first inner layer and the outer layer.

In another preferred embodiment, the processing of steps (a), (b) and (c) can be performed independently and continuously, one by one separately, or by combining both.

Lastly, a third aspect of the invention relates to the cosmetic and/or pharmaceutical use of the patch of the invention as defined above, and particularly for use in the prolonged release of one or more perfumes.

In the present invention, the word "perfume" refers to a substance or mixture of substances, free of impurities or not, that are used to provide a desired odour. The perfume can include essences, additives, solvents and any other type of components including bioactive agents and/or other cosmetic or pharmaceutical substances, typically used by the industry.

The formulation may contain chemical compounds that fall within the composition of the perfume base such as, for example, and without implying any limitation: acetophenone, methylacetophenone, cinnamic aldehyde, amyl cinnamic, lanisic, cumin, cyclamen, hydratropic, p-cresyl methyl ether, benzophenone, citral, citronellyl oxyacetaldehyde, allyl hexanoate, amyl hexanoate, cinnamyl isobutyrate, geranyl acetates, linalyl acetate or phenylacetate, menthyl, phenylethyl, vetiveryl, jasmyle, phenylethyl ethyl methyl phenyl glycidate formate, eugenol, isoeugenol, geraniol, citronellal, hydroxycitronellal, ionone, methyl ionone la, phenylacetaldehyde dimethyl acetal, menthol, musk, phenylethyl alcohol, derivatives of pinene and camphene, carvone, cinnamyl alcohol, coumarin, dimethylbenzylcarbinyl acetate, heliotropine, isocyclocitral, methyl nonyl acetaldehyde, undecalactone, vanillin, or any of the possible mixtures of the mentioned compounds.

In another preferred embodiment, the inner layer can alternatively be formed by one or more common and/or commercial adhesive and preferably hypoallergenic materials which are not necessarily manufactured by means of electro-hydrodynamic or aero-hydrodynamic processing techniques. Instead, they can be manufactured using traditional methods, for example, and without limitation, by means of casting, extrusion from the melt, coating, lamination, spraying, etc. This layer, in turn, can be made up of several peelable or non-peelable films, having at least one hypoallergenic adhesive on the side in contact with the skin. This layer can be a double-sided adhesive, wherein there is a hypoallergenic adhesive or not in contact with the intermediate or outer layer and a hypoallergenic adhesive in contact with the skin. Among the adhesives that can be selected, without limitation, are all those already existing in the industry of adhesives, which are hypoallergenic and even more preferably those that are bio-based, soluble in water and/or alcohols or biodegradable. The possible adhesives used can be selected, without limitation, from those of synthetic origin such as those based on acrylic polymers, synthetic rubbers, latex-type materials, epoxy systems, silicones, or polyurethanes, and, on the other hand, adhesives of bio-based origin or bio-adhesives which are formed by variations of lipids, proteins and polysaccharides such as fibrin, hydrogels, chitosans, chitins, carraghenates, alginates, etc. This layer can also be formed by combining the mentioned adhesives with each other and/or together with polymer films, without limitation, from the family of polyolefins, celluloses, fluoropolymers, and/or polyesters, which can serve as a support for the adhesive(s). Alternatively, in the event that the adhesives used require supporting material, this can ideally also be biodegradable or oxobiodegradable, being able to be found among type A and B polymers.

Throughout the description and the claims, the word "comprises" and its variants do not intend to exclude other technical features, additives, components or steps. For those skilled in the art, other objects, advantages and features of the invention may be partially deduced from both the description and the embodiment of the invention. The following examples and figures are provided by way of illustration and are not intended to limit the present invention.

### BRIEF DESCRIPTION OF THE FIGURES

**Fig. 1** shows a general diagram that shows different examples of formulations of patches of the invention.
**Fig. 2** shows a general patch diagram consisting of two layers: total patch (1), which has the layer with Perfume (Polymer B + Perfume, 2) and the layer in contact with the skin (Polymer A, 3).
**Fig. 3** shows a production diagram of a bilayer patch by co-deposition of PCL and PVP/Perfume on a PEO layer, and the corresponding morphologies thereof taken with scanning electron microscopy (SEM).

### EXAMPLES

Next, the invention will be illustrated by means of assays carried out by the inventors which demonstrate the effectiveness of the product of the invention. To do this for each example, a hedonistic qualitative scale established by the perfumer from 1 to 5, 1 being the worst and 5 being the best, is indicated for the features of odour intensity, odour persistence and stickiness of the patch on the skin.

### Example 1: PCL/Perfume-PVP bilayer structure system laminated by means of monoaxial electrospinning

### Preparation of the first inner hydrophilic layer

This layer was made of polyvinylpyrrolidone (PVP). To do this, a solution of PVP at 10% by weight in ethanol was first used. Once dissolved, the fibre sheet is then manufactured using the electrospinning technique. To do this, an emitter voltage of 25kV and a collector voltage of -25kV were used; a flow rate of 45 ml/h was also used, through a 22G multi-outlet linear injector. The fibres were deposited on a rotating collector (100 rpm) coated by a waxed paper substrate and at a distance of 24 cm. The manufacture was carried out at a temperature of 30°C and a relative humidity of 30%. This layer has a surface density of 50 g/m².

### Preparation of the second outer hydrophobic layer with perfume

A solution of Poly-ε-caprolactone (PCL) at 12% by weight, in 79% by weight of Chloroform, 3.6% by weight of Methanol and 5.4% by weight of Perfume supplied by Perfumes Loewe S.A., is made Once dissolved, the fibre sheet is then manufactured using the electrospinning technique. To do this, an emitter voltage of 25kV and a collector voltage of -10kV were used; a flow rate of 50 ml/h was also used, through a 22G multi-outlet linear injector. The fibres were deposited on a rotating collector (100 rpm) covered with a waxed paper substrate and at a distance of 26.5 cm. The manufacture was carried out at a temperature of 30°C and a relative humidity of 30%. This layer has a surface density of 50 g/m².

Both layers were processed in a Fluidnatek LE-100 equipment with an environmental control unit. Once the two layers are available, they are laminated by means of calendering at a speed of 2.56 rpm and a temperature of 52°C.

The features of the patch indicated in this example are shown in the following table:

| **Feature** | **Qualitative value** |
|---|---|
| **Odour intensity** | 2 |
| **Odour persistence** | 3 |
| **Adhesion to skin** | 2 |

### Example 2: PCL/Perfume-PVP/PAC bilayer structure system laminated by means of monoaxial electrospinning

### Preparation of the first inner hydrophilic layer

A solution of 10% by weight of polyvinylpyrrolidone (PVP) and 5% by weight of Polyacrylate (PAC) in ethanol was used. The manufacturing conditions used were the same as those described in Example 1.

### Preparation of the second outer hydrophobic layer with perfume

It was manufactured using the same conditions described in Example 1.

The features of the patch indicated in this example are shown in the following table:

| **Feature** | **Qualitative value** |
|---|---|
| **Odour intensity** | 2 |
| **Odour persistence** | 3 |
| **Adhesion to skin** | 3 |

### Example 3: PCL/Perfume-PEO/PVP/EC bilayer structure system laminated by means of monoaxial electrospinning.

### Preparation of the first inner hydrophilic layer

A solution of 8% by weight of polyethylene oxide (PEO), 4% by weight of polyvinylpyrrolidone (PVP) and 1% by weight of ethyl cellulose (EC) in a mixture of ethanol/water in a ratio of 5:5 was used. The manufacturing conditions used were an emitter voltage of 30V and a collector voltage of -20kV; a flow rate of 26 ml/h was also used, through a 22G multi-outlet linear injector. The fibres were deposited on a rotating collector (200 rpm) covered with a waxed paper substrate and at a distance of 28 cm. The manufacture was carried out at a temperature of 25°C and a relative humidity of 30%. In this case the surface density is 100 g/m².

### Preparation of the second outer hydrophobic layer with perfume

It was manufactured using the same conditions described in Example 1, but in this case the surface density is 300 g/m²

The features of the patch indicated in this example are shown in the following table:

| **Feature** | **Qualitative value** |
|---|---|
| **Odour intensity** | 3 |
| **Odour persistence** | 3 |
| **Adhesion to skin** | 4 |

### Example 4: PCL-PVP/Perfume-PEO three-layer system laminated by means of continuous monoaxial electrospinning.

This example is composed of three layers, which are joined together using the continuous multilayer manufacturing method followed by laminating, in other words, each layer is deposited on top of the preceding one continuously in a single step.

### Preparation of the first inner hydrophilic layer

This layer was made of polyethylene oxide (PEO). To do this, a solution of PEO at 13% by weight in ethanol and water in a 7:3 ratio was first used. To do this, an emitter voltage of 20kV and a collector voltage of -10kV were used; a flow rate of 52 ml/h was also used, through a 22G multi-outlet linear injector. The fibres were deposited on a rotating collector (100 rpm) covered with a waxed paper substrate and at a distance of 28 cm. The manufacture was carried out at a temperature of 30°C and a relative humidity of 30%. This layer has a surface density of 30 g/m².

### Preparation of the second intermediate hydrophilic layer with perfume

A solution of PVP at 20% by weight in the Perfume is made, in other words, the Perfume acts as a solvent. This layer is manufactured at the end of the preceding one. To do this, an emitter voltage of 20kV and a collector voltage of -10kV were used; a flow rate of 26 ml/h was also used, through a 22G multi-outlet linear injector. The fibres were deposited on the PEO layer in the same rotating collector (100 rpm) but at a distance of 20 cm, with the intention that the fibres arrive wet enough for the PEO layer to adhere. The manufacture was carried out at a temperature of 30°C and a relative humidity of 30%. This layer has a surface density of 80 g/m².

### Preparation of the third outer hydrophobic layer

A solution of Poly-ε-caprolactone (PCL) at 12% by weight, in 79% by weight of Chloroform and 9% Methanol is made. To produce this layer on the PVP/Perfume layer, an emitter voltage of 23kV and a collector voltage of -1kV were used; a flow rate of 10 ml/h was also used, through a 22G multi-outlet linear injector. This last layer must have a surface density between 2 g/m² since the main function thereof is protection, and as a base for logo printing

Finally, lamination of the patch is carried out to further promote adhesion between the layers and offer a smoother appearance. This process is carried out at a temperature of 40°C and at a roller speed of 13 rev/min.

The three layers were processed on a Fluidnatek LE-100 equipment, each one followed by the preceding one. Once the production is completed, this three-layer system is maintained at 30°C and 30% relative humidity inside the equipment, with the intention of preventing possible delamination due to rapid drying of the layers. The manufacture was carried out at a temperature of 25°C and a relative humidity of 30% on a Fluidnatek LE-100 equipment.

The features of the patch indicated in this example are shown in the following table:

| **Feature** | **Qualitative value** |
|---|---|
| **Odour intensity** | 4 |
| **Odour persistence** | 4 |
| **Adhesion to skin** | 4 |

### Example 5: PCL/Perfume-PVP/Perfume-PEO three-layer system by means of continuous monoaxial electrospinning.

This example is the same as the preceding one manufactured by continuous multilayer manufacturing and laminating, but in this case the PCL outer layer also contains the same perfume.

The latter is carried out with a solution of Poly-ε-caprolactone (PCL) at 12% by weight, in 79% by weight of Chloroform, 3.6% by weight Methanol and 5.4% by weight of perfume. Once dissolved, the manufacturing process is carried out on the PVP/Perfume layer. To do this, an emitter voltage of 25kV and a collector voltage of -10kV were used; a flow rate of 50 ml/h was also used, through a 22G multi-outlet linear injector. The fibres were deposited on a rotating collector (100 rpm) covered with a waxed paper substrate and at a distance of 26.5 cm. The manufacture was carried out at a temperature of 30°C and a relative humidity of 30%. This layer has a surface density of 5 g/m².

Subsequently, calendering is carried out at 40°C at a speed of 13 rpm. In this way the adhesion between layers is ensured.

The manufacture was carried out at a temperature of 25°C and a relative humidity of 30% on a Fluidnatek LE-500 equipment.

The features of the patch indicated in this example are shown in the following table:

| **Feature** | **Qualitative value** |
|---|---|
| **Odour intensity** | 5 |
| **Odour persistence** | 5 |
| **Adhesion to skin** | 4 |

### Example 6: PCL-PVP/Perfume-PCL-PEO four-layer system laminated by means of continuous monoaxial electrospinning.

This example is the same as the preceding one manufactured by continuous multilayer manufacturing and laminating, but in this case an intermediate layer is added between the PEO layer and the PVP/Perfume layer. The function of this is to improve adhesion between layers and act as a moisture barrier.

This layer is made of Poly-ε-caprolactone (PCL), which acts as an adhesive and a perfume barrier, so that it does not spread to the side of the user's skin. To make this layer, a solution of Poly-ε-caprolactone (PCL) at 12% by weight, in 79% by weight of Chloroform and 9% Methanol was used. To produce this layer on the PEO layer, an emitter voltage of 23kV and a collector voltage of -1 kV were used; a flow rate of 10 ml/h was also used, through a 22G multi-outlet linear injector. This very last layer must have a surface density between 2 g/m².

The PVP/Perfume layer is deposited on this layer as indicated in the preceding example. And this in turn would be coated by a last layer of PCL, also indicated in the preceding case.

Once production is completed, this four-layer system is maintained at 30°C and 30% relative humidity inside the equipment, with the intention of preventing possible delaminations due to rapid drying of the layers. Subsequently, calendering is carried out at 40°C at a speed of 2.56 rpm. In this way the adhesion between layers is ensured.

The manufacture was carried out at a temperature of 25°C and a relative humidity of 30% on a Fluidnatek LE-500 equipment.

The features of the patch indicated in this example are shown in the following table:

| **Feature** | **Qualitative value** |
|---|---|
| **Odour intensity** | 4 |
| **Odour persistence** | 4 |
| **Adhesion to skin** | 4 |

### Example 7: PCL/Perfume-PEO/EC/Perfume-PCL/Perfume-PEO/PVP/EC-PCL-PEO/PVP/EC six-layer system laminated by means of monoaxial and continuous electrospinning.

This example is the same as the preceding one, manufactured by continuous multilayer manufacturing and laminating, but in this case the system of materials set out in Example 3 is used. In this case, different hydrophobic layers are interspersed, the function of which, in addition to improve the adhesion between layers, is to act as a moisture barrier which prevents the transparency of the patch promoted by the complete dissolution of the hydrophilic layer. This patch concept comprises the following layers:

### Preparation of the first inner hydrophilic layer

This layer was made of a mixture of Polyethylene oxide/Polyvinylpyrrolidone/Ethyl cellulose using the same conditions described in Example 3, but with a surface density of 175 g/m².

### Preparation of the second intermediate hydrophobic barrier layer

This layer was made of Poly-ε-caprolactone rover the first layer using the same conditions showed in Example 4.

### Preparation of the third intermediate hydrophilic layer

This layer was made over the previous layer using the same conditions as the first layer, but with a surface density of 40 g/m².

### Preparation of the fourth intermediate hydrophobic barrier layer with perfume

This layer was made of Poly-ε-caprolactone (PCL) with perfume on the preceding layer using the conditions described in Example 1.

### Preparation of the fifth intermediate hydrophilic layer with perfume.

This layer is the one that storages most of the perfume and for this a solution at 8% by weight of Polyethylene oxide and 3% by weight of Ethyl cellulose was made in a Perfume/Water mixture in a weight ratio of 7:3. As with the preceding layers, this was also produced on the preceding layer using an emitter voltage of 30kV and a collector voltage of -30kV, a flow rate of 26 ml/h was also used, through a 22G multi-outlet linear injector. The fibres were deposited on a rotating collector (100 rpm) coated by a waxed paper substrate and at a distance of 28 cm. This layer has a surface density of 360 g/m².

### Preparation of the sixth outer hydrophobic barrier layer with perfume

This layer was made of Poly-ε-caprolactone (PCL) with perfume on the preceding one using the conditions described in Example 1, and on the preceding layer with a surface density of 50 g/m².

The manufacture was carried out at a temperature of 25°C and a relative humidity of 30% on a Fluidnatek LE-500 equipment. Once the last layer is completed. Once the production of the six-layer system is completed, calendering is carried out at 40°C at a speed of 2.56 rpm. In this way the adhesion between layers is ensured.

The features of the patch indicated in this example are shown in the following table:

| **Feature** | **Qualitative value** |
|---|---|
| **Odour intensity** | 4 |
| **Odour persistence** | 3 |
| **Adhesion to skin** | 3 |

### Example 8: Bilayer system laminated by means of co-deposition on PEO fibres of PCL and PVP/Perfume fibres by means of electrospinning.

As in the preceding examples, this example is made up of a PEO layer, which is the one that adheres to the skin. This is manufactured in the same way as in the preceding examples. This layer has a surface density of 30 g/m².

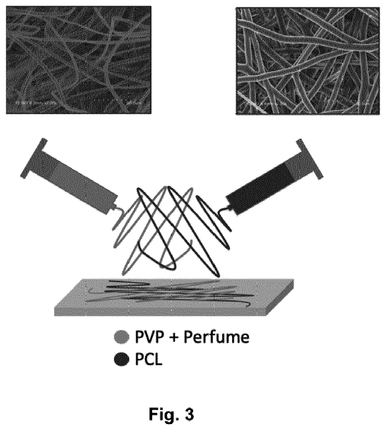

Then, a second layer formed by PVP fibres with Perfume and PCL fibres is deposited on the same, acting as a barrier to, in addition to the release of the perfume, increase the persistence thereof (see diagram Figure 3). First, the polyethylene oxide PEO fibres are deposited. To do this, a solution of PEO at 13% by weight in ethanol and water in a 7:3 ratio was first used. To do this, an emitter voltage of 20kV and a collector voltage of - 10kV were used; a flow rate of 52 ml/h was also used, through a 22G multi-outlet linear injector. The fibres were deposited on a flat collector, which moved with a roll-to-roll system, and was covered with a waxed paper substrate at a distance of 28 cm. The manufacture was carried out at a temperature of 30°C and a relative humidity of 30%.

Then after a deposition is carried out, the solution of PVP with perfume is deposited with an adjacent injector. To do this, an emitter voltage of 20kV and a collector voltage of - 10kV were used; a flow rate of 26 ml/h was also used, through a 22G multi-outlet linear injector. The fibres were deposited simultaneously with the PCL fibres which are produced using another contiguous injector. For this second deposition, a solution of Poly-ε-caprolactone (PCL) at 12% by weight, in 79% by weight of Chloroform and 9% Methanol is made. To produce this layer on the PVP/Perfume layer, an emitter voltage of 20kV and a collector voltage of -10kV were used; a flow rate of 10 ml/h was also used, through a 22G multi-outlet linear injector.

This layer made of a combination of PVP/Perfume and PCL fibres has a surface density of 80 g/m²

The roll-to-roll system enables the substrate to pass under both injectors continuously, in other words, PCL and PVP/Perfume depositions are simultaneously carried out. Both layers were processed on a Fluidnatek LE-500 equipment. Once production is completed, this system is kept at 30°C and 30% RH inside the equipment.

The manufacture was carried out at a temperature of 25°C and a relative humidity of 30% on a Fluidnatek LE-500 equipment.

The features of the patch indicated in this example are shown in the following table:

| **Feature** | **Qualitative value** |
|---|---|
| **Odour intensity** | 3 |
| **Odour persistence** | 5 |
| **Adhesion to skin** | 4 |

### Example 9: Continuous three-layer system of PVP core-shell fibres with Perfume, between PEO and PCL layers

As in the preceding examples, this example is made up of a first PEO layer, which is the one that adheres to the skin. This is manufactured in the same way as in the preceding examples. This layer has a surface density of 30 g/m².

In this case, for the second intermediate layer, a device made up of coaxial nozzles is used. A solution of PVP in ethanol at 20% by weight is injected through the outer nozzle (Shell G14), using an emitter voltage of 20kV and a collector voltage of -10kV; a flow rate of 26 ml/h was also used. This results in the formation of tubular fibres. Perfume is introduced through the inner nozzle (Core G27), such that the perfume is encapsulated in the tubular fibres. The flow rate injected is 10 ml/h, obviously the rest of the parameters are the same since it is the same nozzle holder. This layer has a surface density of 50 g/m².

Lastly, a third outer layer PCL is added to keep the perfume, as in the preceding cases, using another injector. This layer has a surface density of 2 g/m².

The manufacture was carried out at a temperature of 25°C and a relative humidity of 30% on a Fluidnatek LE-100 equipment.

The features of the patch indicated in this example are shown in the following table:

| **Feature** | **Qualitative value** |
|---|---|
| **Odour intensity** | 2 |
| **Odour persistence** | 5 |
| **Adhesion to skin** | 4 |

### Example 10: Continuous multilayer system of PLA fibres filled with encapsulated perfume particles.

As in the preceding examples, this example is made up of a first PEO layer, which is the one that adheres to the skin. This is manufactured in the same way as in the preceding examples. This layer has a surface density of 30 g/m².

In this case, the perfume is encapsulated in nanoparticles of whey protein, manufactured by means of air-assisted electrospray with a Capsultek equipment from Bioinicia S.L. The polymer was dissolved in water at 20% and the perfume to which the surfactant TEGO SML from Evonik at 9% was added was slowly added. The system was homogenised using an UltraTurrax T-25 homogeniser (IKA, Staufen, Germany) at 17,000 rpm for 5 min, followed by 5 minutes of ultrasound (Bandelin Sonopuls, Berlin, Germany) to achieve a 2:1 polymer/perfume ratio. Particles of 3 microns were obtained by processing the solution in the Capsultek equipment at 25°C and 30% RH at a rate of 1 ml/min with a voltage of 10 kV and a carrier gas flow of 10 l/min. These particles are added in a solution of PLA at 10% in 79% by weight of chloroform. From this solution, the second layer of fibres is deposited on the PEO layer, using a voltage, flow rate of 25 ml/h, at a height of 20 cm. The deposition was carried out at a temperature of 25°C and a relative humidity of 30%. The function of the PLA is to retain the release of the perfume from the nanoparticles, in such a way that the odour is retained even longer over time. This layer has a surface density of 60 g/m². The manufacture was carried out at a temperature of 25°C and a relative humidity of 30% on a Fluidnatek LE-100 equipment.

The features of the patch indicated in this example are shown in the following table:

| **Feature** | **Qualitative value** |
|---|---|
| **Odour intensity** | 1 |
| **Odour persistence** | 5 |
| **Adhesion to skin** | 4 |

### Example 11: PEO-PVP/Perfume-PCL continuous three-layer system produced by means of solution blow spinning.

In this example, all the layers are manufactured by means of the solution blow spinning technique.

As in the preceding examples, this example is made of a first PEO layer, which is the one that adheres to the skin. This is manufactured by using a solution at 10% by weight of PEO in a Chloroform/Acetone mixture in a ratio of 8:2 by weight. For this, a coaxial injector was used, through which the outer nozzle (1 mm in diameter) circulates an air flow at a pressure of 0.5 bar, while through the inner nozzle (0.5 mm in diameter) the polymer solution circulates with a flow rate of 15 ml/h. These fibres are deposited on a rotating collector (120 rpm) located 20 cm from the nozzle. This layer has a surface density of 70 g/m².

The second layer, as in the preceding examples, is the one that contains the perfume. This layer is made with a solution of PVP at 10% by weight in perfume. For this, a pressure of 0.5 bar and a flow rate of 10 ml/h were used through a coaxial injector. The fibres were deposited on the PEO layer at a distance of 10 cm. This layer has a surface density of 100 g/m².

Lastly, a third layer of PCL was deposited. For this, a solution of PCL at 12% by weight, in 79% by weight of Chloroform and 9% Methanol was used. To produce this layer on the PVP/Perfume layer, a pressure of 1 bar and a flow rate of 10 ml/h were used through a coaxial injector. This layer was deposited at a distance of 10 cm. This very last layer must have a surface density between 2 g/m² since the main function thereof is protection, and as a base for logo printing.

The manufacture was carried out at a temperature of 25°C and a relative humidity of 30% on a Fluidnatek LE-100 equipment.

The features of the patch indicated in this example are shown in the following table:

| **Feature** | **Qualitative value** |
|---|---|
| **Odour intensity** | 4 |
| **Odour persistence** | 5 |
| **Adhesion to skin** | 4 |

### Example 12: Continuous three-layer system produced by means of emulsion electrospinning.

As in the preceding examples, this example is made of a first PEO layer, which is the one that adheres to the skin. This is manufactured in the same way as in the preceding examples. This layer has a surface density of 30 g/m².

In this case, for the second layer, a solution of PEO at 13% by weight in water is first used and the perfume with 9% TEGO SML surfactant is added little by little to have a 2:1 polymer:perfume emulsion. The system was homogenised using an UltraTurrax T-25 homogeniser (IKA, Staufen, Germany) at 17,000 rpm for 5 min, followed by 5 minutes of ultrasound (Bandelin Sonopuls, Berlin, Germany) and processed by means of electrospinning. To do this, an emitter voltage of 20kV and a collector voltage of -10kV were used; a flow rate of 52 ml/h was also used, through a 22G multi-needle linear injector. The fibres were deposited on a rotating collector (100 rpm) coated by a waxed paper substrate and at a distance of 28 cm. This layer has a surface density of 60 g/m2.

Lastly, a third layer of PCL is added to keep the perfume, as in the preceding cases, using another injector. This layer has a surface density of 2 g/m².

The manufacture was carried out at a temperature of 25°C and a relative humidity of 30% on a Fluidnatek LE-100 equipment.

The features of the patch indicated in this example are shown in the following table:

| **Feature** | **Qualitative value** |
|---|---|
| **Odour intensity** | 3 |
| **Odour persistence** | 5 |
| **Adhesion to skin** | 4 |

### Example 13: PCL/Perfume-Commercial adhesive bilayer structure system with peelable double side.

This example comprises two layers, a first inner layer based on a hypoallergenic double-sided commercial adhesive tape for contact with the skin, on which the PCL/Perfume outer layer is electrospun.

A hydrophobic perfume-containing layer made of PCL is deposited on this double-sided adhesive. To produce this layer, a solution at 12% by weight of PCL was used in a Chloroform/Perfume mixture in a ratio of 7:3 by weight. To produce this one, an emitter voltage of 20kV and a collector voltage of -5kV were used; a flow rate of 26 ml/h was also used, through a 22G multi-outlet linear injector. The fibres were deposited on the adhesive layer in a rotating collector (200 rpm) at a distance of 25 cm. The manufacture was carried out at a temperature of 25°C and a relative humidity of 30%. This layer has a surface density of 200 g/m². Once the production of this system was completed, calendering was carried out at 25°C with a speed of 2.56 rpm. In this way the adhesion between layers is increased.

| **Feature** | **Qualitative value** |
|---|---|
| **Odour intensity** | 5 |
| **Odour persistence** | 5 |
| **Adhesion to skin** | 5 |

## Claims

1. A perfume-emitting patch **characterised in that** it comprises at least:
i) an inner layer that is in contact with the user's skin to which it adheres, **characterised in that** it is made up of fibres of a polymer A, and **in that** it has a surface density of at least 0.1 g/m²;
ii) a series of between 0 and 1000 consecutive intermediate layers deposited on the inner layer, **characterised in that** they are independently made up of fibres of a polymer A or B, or mixtures of both, and **in that** it has a surface density of at least 0.1 g/m²;
iii) an outer layer **characterised in that** it is composed of fibres of a polymer B, and **in that** it has a surface density of at least 0.1 g/m²,
wherein at least one of the intermediate layers and/or the outer layer contains the encapsulated perfume.

2. The patch according to the preceding claim, wherein the polymer A is selected from among polyethylene oxide and derivatives thereof as non-ionic water-soluble resins, polyvinylpyrrolidone and the copolymers thereof, polyvinyl alcohols and the copolymers thereof with ethylene, polyacrylates, polyacrylic acid, water-soluble polyacronitriles, lignin and derivatives such as sulfonated lignin, acrylic/methacrylic ester polymers, polysaccharides and derivatives, such as for example pullulan, hyaluronic acid, alginate, tragacanth, carrageenan, chitin and derivatives such as chitosan, celluloses such as ethylcellulose, hydroxypropylcellulose, methylcellulose, or hydroxypropylmethylcellulose, gluocogen, starch and polymers derived from the same such as thermoplastic starch, pectin, guar, xanthan, fructosan or gellan among others, proteins and derivatives, such as collagen, gelatin, soy protein, whey protein, zein, gluten, casein, or lectins among others, thiolated polymers, polyanhydrides, and PAA polyethylene glycol copolymers, as well as the mixtures thereof.

3. The patch according to the preceding claim, wherein the polymer A of the inner layer that is in contact with the skin is selected from polyvinylpyrrolidone, polyethylene oxide, polyvinyl alcohols and polyacrylate, or combinations thereof.

4. The patch according to any of the preceding claims, wherein the polymer A of the inner layer that is in contact with the skin is polyethylene oxide.

5. The patch according to any of claims 1 to 3, wherein the polymer A of the inner layer that is in contact with the skin is polyvinylpyrrolidone.

6. The patch according to any of claims 1 to 3, wherein the polymer A of the inner layer that is in contact with the skin is a mixture of polyvinylpyrrolidone and polyacrylate.

7. The patch according to any of claims 1 to 3, wherein the polymer A of the inner layer that is in contact with the skin is a mixture of polyethylene oxide, polyvinylpyrrolidone and ethyl cellulose.

8. The patch according to any of the preceding claims, wherein the inner layer is formed by at least one adhesive material.

9. The patch according to the preceding claim, wherein the adhesive material is hypoallergenic.

10. The patch according to any of claims 8 to 9, wherein the adhesive material is not manufactured by electro-hydrodynamic or aero-hydrodynamic processing techniques.

11. The patch according to any of the preceding claims, wherein the polymer B is selected from non-water-soluble proteins such as keratin, waxes or paraffins, polyhydroxyalkanoates such as PHB, PHV, medium chain length PHA, and all the possible copolymers thereof such as PHBV among others, poly-ε-caprolactone and all the copolymers thereof such as PEG-PCL and PCLA, polylactic acid, all the copolymers thereof such as PGLA, polyphosphazenes, polyorthoesters, polyesters obtained starting from natural precursors such as polytrimethylene terephthalate, polybutylene terephthalate, polybutylene succinate, and all the possible copolymers thereof such as poly(butylene adipate-co-terephthalate), polyethylene-co-vinyl acetate, polyethylene terephthalate and copolymers thereof, silicones, polyesters, polyurethanes, polysulfones, halogenated polymers such as polyvinylidene fluoride or polyvinylidene chloride, polycarbonates, acrylonitrile butadiene styrene, latex, and polyamides such as 6 Nylon 6, Nylon 66 or Nylon 69, as well as the mixtures thereof.

12. The patch according to the preceding claim, wherein the polymer B of the outer layer is poly-ε-caprolactone, poly-ε-caprolactone copolymers, or any of the mixtures thereof.

13. The patch according to any of the preceding claims, wherein the patch comprises one, two, three or four intermediate layers between the inner layer and the outer layer.

14. The patch according to the preceding claim, wherein the polymer of the intermediate layers is independently selected from poly-ε-caprolactone, poly-ε-caprolactone copolymers, polyvinylpyrrolidone, polyethylene oxide and ethyl cellulose, or any of the mixtures thereof.

15. The patch according to any of claims 13 to 14, wherein at least one of the intermediate layers between the hydrophilic inner layer that is in contact with the user's skin and the hydrophobic outer layer contains perfume.

16. The patch according to the preceding claim, wherein the outer layer does not contain perfume.

17. The patch according to any of the preceding claims, wherein the perfume contained in the intermediate layers contains the same perfume as the hydrophobic outer layer.

18. A method for obtaining a perfume-emitting patch according to any of claims 1 to 13 comprising the following steps:
a) Preparation of the inner layer starting from a solution of the polymer A at a concentration between 0.01 and 98% by weight, wherein the emitter voltage used is between 0.01 and 500kV and a collector voltage between 0 kV and -500kV, with a flow rate between 0.0001 and 1,000,000 ml/h, at a temperature between 1°C and 100°C and a relative humidity between 0% and 100%;
b) Preparation of the intermediate layers located between the inner layer and the outer layer starting from a solution of the polymer A or B at a concentration between 0.01 and 98% by weight, and of a perfume in a concentration between 0 and 98% by weight, wherein the emitter voltage used is between 0.01kV and 500kV and the collector voltage is between 0kV and -500kV, with a flow rate between 0.0001 and 1,000,000 ml/h, at a temperature between 1°C and 100°C and a relative humidity between 0% and 100%;
c) Preparation of the outer layer starting from a solution of the polymer B at a concentration between 0.01 and 98% by weight, and of a perfume in a concentration between 0 and 98% by weight, wherein the emitter voltage used is between 0.01kV and 500kV and the collector voltage is between 0.01kV and - 500kV, with a flow rate between 0.0001 and 1,000,000 ml/h, at a temperature between 1°C and 100°C and a relative humidity between 0% and 100%;
d) Processing of the layers produced in steps (a), (b), and (c) by means of calendering in a speed range between 0.001 and 100,000 rpm and a temperature between 5 and 300°C.

19. The method according to the preceding claim, wherein the processing of steps (a), (b) and (c) is performed independently and continuously, one by one separately, or by combining both.

20. A cosmetic and/or pharmaceutical use of the patch according to any of claims 1 to 17 for the prolonged release of one or more perfumes.
